# FASCICULE DE BREVET EUROPEEN

(11) **EP 1 910 411 B1**
(45) Date de publication et mention de la délivrance du brevet: **03.08.2016**
(21) Numéro de dépôt: 06794299.5
(22) Date de dépôt: 07.08.2006
(51) Int. Cl.: C07K 14/415, A61K 36/48, A01N 25/10, A61Q 5/02, A61K 8/64, A61Q 5/12, A61Q 19/10, C10M 149/14, C02F 1/54

(54) **PRODUIT ISSU D'UN EXTRAIT PROTÉINIQUE DE GUAR, PROCÉDÉ DE PREPARATION ET UTILISATIONS**
GUAR PROTEINEXTRAKTE UND DEREN VERWENDUNG
GUAR PROTEIN EXTRACT PRODUCT AND METHOD FOR THE PRODUCTION AND USE THEREOF

(30) Priorité: 05.08.2005 FR 0508380
(43) Date de publication de la demande: 16.04.2008
(73) Titulaire: RHODIA CHIMIE, 93300 Aubervilliers (FR)
(72) Inventeur: KARAGIANNI, Katerina, F-75013 Paris (FR); MONIN, Vincent, Plainsboro, NJ 08536 (US); SASSI, Jean-François, F-42800 Saint-Romain en Jarez (FR)
(74) Mandataire: Cardon, Flavie
(86) Numéro de dépôt international: PCT/FR2006/001913
(87) Numéro de publication internationale: WO 2007/017590

(56) Documents cités:
- WO-A-01/93810
- WO-A-89/07435
- FR-A- 2 437 829
- KHALIL M M: "BIOCHEMICAL AND TECHNOLOGICAL STUDIES ON THE PRODUCTION OF ISOLATED GUAR PROTEIN" DIE NAHRUNG, VCH VERLAGSGESELLSCHAFT, WEINHEIM, vol. 45, no. 1, 2001, pages 21-24, XP008021585 ISSN: 0027-769X cité dans la demande
- NATH J P ET AL: "Functional properties of guar proteins" JOURNAL OF FOOD SCIENCE, INSTITUTE OF FOOD TECHNOLOGISTS, CHICAGO, IL, US, vol. 46, no. 4, 1981, pages 1255-1259, XP002272557 ISSN: 0022-1147
- TRIMBLE R: "THE POSSIBLE UTILIZATION OF GUAR PROTEIN" PROCEEDINGS INTERNATIONAL CONGRESS FOOD SCIENCE AND TECHNOLOGY, vol. 1, 1983, pages 29-39, XP008028375
- NATH J P ET AL: "Effect of Detoxification Treatments on the Proteins of Guar Meal", JOURNAL OF AGRICULTURAL AND FOOD CHEMISTRY, AMERICAN CHEMICAL SOCIETY, US, vol. 29, no. 3, 1 May 1981 (1981-05-01), pages 529-532, XP001350105, ISSN: 0021-8561

## Description

La présente invention a pour objet des produits issus d'un extrait protéinique de guar modifié, leur procédé de préparation et les compositions cosmétiques, pharmacologiques, phytosanitaires ou pour soin ménager les comprenant. Ces produits et leurs formulations présentent notamment un intérêt particulier dans le domaine de la cosmétique, notamment pour la réalisation de produits de coiffage, pour la mise en forme des cheveux, ou pour la réalisation de shampoings, après-shampoings ou gels douches, pour le conditionnement de la peau et/ou des cheveux. Ils présentent également un intérêt dans le domaine de la détergence, notamment pour les soins domestiques.

L'utilisation d'extraits de guar est connue. Par exemple, on connaît l'utilisation de gomme de guar ou de dérivés de guar dans les domaines de la cosmétique et de l'alimentaire, notamment comme agent de modification de la rhéologie et/ou de la texture d'une composition ou d'un aliment, ou comme agent conditionneur sur la peau et/ou les cheveux.

Les fractions de protéines contenues dans la gomme de guar ou dans les farines d'extraction de guar ont également été décrites (Anderson et al., Food Additives and Contaminants, 1985, vol. 2, N°4, 225-230 ; Nath et al., J. Agric. Food Chem., 1980, 28, 844-847). M. M. Khalil, (Production of Isolated Guar Protein, Food 45, 2001, N° 1, 21-24) s'est plus particulièrement intéressé aux qualités nutritionnelles des protéines isolées à partir de farines de graines de guar ("guar seed flour").

Il existe par ailleurs dans l'industrie un besoin constant pour de nouvelles compositions, par exemple comprenant de nouveaux produits, pouvant présenter de nouvelles propriétés ou améliorer des propriétés. En particulier, il existe un grand intérêt pour les produits d'origine végétale.

Il a maintenant été mis en évidence que, lorsque l'on greffe des groupes chimiques sur les protéines contenues dans l'extrait protéinique de guar, un produit présentant des propriétés bénéfiques sur la peau et les phanères, notamment sur les cheveux, est obtenu. De tels extraits de protéine de guar dérivés sont ainsi particulièrement utiles en cosmétique de soin et en pharmacologie, notamment en dermatologie. Plus spécifiquement, il a été observé que ce produit greffé présente d'excellentes propriétés de conditionnement du cheveu et de la peau. Par ailleurs, l'extrait présente une modularité de formulation (formulabilité) élevée. Cela peut rendre son utilisation simple et peu coûteuse.

Ainsi, il a été observé que l'extrait protéinique de guar cationisé est compatible avec la plupart des tensioactifs anioniques couramment utilisés dans le domaine des produits cosmétiques, pharmacologiques, phytosanitaires et des détergents, pour une large gamme de compositions.

Il a également été montré que ces produits peuvent être utilisés dans les compositions pour les soins ménagers (aussi bien pour les soins mis en oeuvre dans la sphère privée des consommateurs, que pour les soins mis en oeuvre dans une sphère publique comme le nettoyage industriel ou institutionnel des surfaces et textiles), en particulier des compositions détergentes, notamment pour le traitement, par exemple le nettoyage, des surfaces dures, dont la vaisselle, ou des surfaces textiles. De façon plus spécifique, ces compositions permettent d'adoucir et de faciliter le repassage des tissus. Elles permettent également de faciliter le nettoyage des surfaces dures.

De manière inattendue, il a en outre été mis en évidence qu'il était possible de diminuer le rebond des gouttes et d'accroître la rétention des formulations phytosanitaires et/ou d'éléments nutritionnels en introduisant des extraits protéiniques de guar dérivés dans lesdites formulations appliquées à des plantes. Les extraits protéiniques de guar dérivés possèdent un effet positif sur l'adhésion instantanée et, par conséquent, sur la rétention, dans des conditions de granulométrie élevée, des gouttes de pulvérisation. Avantageusement, ces extraits limitent significativement les phénomènes de rebond, usuellement observés lors de la pulvérisation sous forme de gouttes de granulométrie élevée et limitent par ailleurs le phénomène de ruissellement. Les extraits de protéines de guar dérivés améliorent ainsi l'adhésion instantanée et, par conséquent, la rétention phytosanitaire et/ou d'éléments nutritionnels appliqués sous forme de gouttelettes sur les plantes à traiter.

### Définitions

Dans la présente demande, on désigne par guar la plante *Cyanopsis tetragonoloba.* Dans le présent exposé, les pourcentages en poids sont exprimés en poids sec, sauf mention contraire.

Dans la présente demande on désigne par "graines de guar" ("guar seeds" en anglais) les graines issues du guar. Les graines de guar comprennent l'écorce ("Hull" en anglais), plus ou moins fibreuse, le germe, et deux cupules de quar ("guar splits" on "endosperme halves" en anglais) qui constituent l'endosperme de guar. Les cupules (respectivement l'endosperme) sont (est) riche(s) en galactomannes. Les graines de guar sont constituées généralement de 35 à 40% en poids de l'endosperme, de 42 à 47% en poids du germe, et de 14 à 17 % en poids de l'écorce.

Dans la présente demande, on désigne par "farine de guar" ("guar flour" en anglais) ou par poudre de guar ("guar powder" en anglais), une poudre issue de l'endosperme de guar.

Dans la présente demande, on désigne par "guar natif" des chaînes macromoléculaires de type galactomannane issues de l'endosperme de guar, n'ayant pas subi de modification chimique par greffage de groupes chimiques. Le guar natif comprend des macromolécules comportant une chaîne principale d'unités D-mannopyranose liées en position bêta (1-4) substituée par des unités D-galactopyranose en position bêta (1-6). Le guar natif présente un rapport mannose/ galactose d'environ 2. Le guar natif peut éventuellement avoir été partiellement dépolymérisé (abaissement de la masse moléculaire). On désigne par "guar dérivé" ("derivatized guar" en anglais) des chaînes macromoléculaires issues de guar natif, ayant subi une modification chimique par greffage de groupes chimiques.

Dans la présente demande, on désigne par "gomme de guar" ("guar gum" en anglais) un produit essentiellement constitué de guar natif, sous forme de cupules guar ("guar splits") ou de farine ou de poudre de guar ("guar flour" ou "guar powder").

Le germe du guar comprend généralement de 35 à 45 % en poids de protéines, de 30 à 35% en poids de fibres, moins de 5% de galactommananes, environ 5% de sels, environ 5-10% d'eau, environ 6% de graisses, les pourcentages en poids étant exprimés par rapport au poids total de germe de guar. Le germe de guar ("churi" dans la langue utilisée dans les bassins de culture de l'Inde et du Pakistan notamment) est parfois désigné de manière impropre sous le terme de "protéine de guar". Dans la présente demande, le terme "protéine de guar," ne désigne pas le germe du guar.

Les cupules de guar comprennent environ 4 à 6% de matière protéinique.

L'écorce de guar ne comprend généralement pas de protéines (environ 0% en poids). L'écorce de guar est parfois désignée par "korma" dans la langue utilisée dans les bassins de culture de l'Inde et du Pakistan notamment.

La gomme de guar est obtenue par un procédé comprenant la séparation plus ou moins fine d'un produit comprenant les cupules de guar d'une part (avec éventuellement des impuretés) et d'un sous-produit comprenant l'écorce et le germe d'autre part (avec éventuellement des impuretés). Le procédé est généralement essentiellement mécanique, mais des étapes de lavage et/ou d'extraction à l'aide d'eau ou de solvants ou d'agents gonflants, ainsi que des étapes de purification avec des agents acides ou basiques peuvent intervenir. Ces procédés, étapes, produits et sous-produits sont connus de l'homme du métier.

Dans la présente demande, on désigne par "farine d'extraction de guar" ("guar meal" en anglais), le sous-produit issu de la récupération des cupules, comprenant typiquement environ 70-80% en poids de germe de guar ("churi"), et environ 20-30% en poids d'écorce ("korma") et moins de 10% en poids d'endosperme.

Dans la présente demande, on désigne par "extrait protéinique de guar" ou "protéine de guar" un produit comprenant au moins 65% en poids de protéines, typiquement de 65 à 95% en poids, extraites du germe de guar, typiquement issu d'un procédé de concentration et/ou d'extraction et/ou d'isolement à partir de farine d'extraction de guar. Dans la présente demande, on pourra également se référer à un "isolat de protéine de guar" ("guar proteine isolate" en anglais) ou à un "concentré de protéine de guar" ("guar proteine concentrate" en anglais).

Dans la présente demande, sauf mention contraire, les quantités en poids de protéines sont déterminées à partir du taux d'azote mesuré selon la méthode Kjeldhal, connue. Cette méthode est par exemple décrite sur le lien suivant: http://www.rosesci.com/Products/Chemical%20Analysis/Kjeldahl%20 Chemistry%20-%20Overview.htm. Le taux d'azote est multiplié par 6,25 pour obtenir la quantité en poids de protéine.

Dans la présente demande, on désigne par "extrait protéinique de guar dérivé" ou "protéine de guar dérivée" un produit susceptible d'être obtenu par modification chimique des molécules de l'extrait protéinique de guar. En d'autres termes, il s'agit d'un produit comprenant des protéines de guar modifiées par des groupes chimiques greffés.

Selon un premier aspect, l'invention a donc pour objet un produit issu d'un extrait protéinique de guar, caractérisé en ce qu'il comprend des groupes chimiques greffés de façon covalente sur des fonctions d'acides aminés compris dans l'extrait protéinique, ledit extrait protéinique étant notamment issu des farines d'extraction de guar.

La composition en acides aminés et la biodistribution de la masse moléculaire de l'extrait protéinique est susceptible de varier de manière plus ou moins importante en fonction de l'origine des graines de guar, de leur maturation, des conditions d'extraction utilisées.

Parmi les acides aminés présents dans l'extrait protéinique de guar, on peut citer principalement l'acide glutamique (Glu), l'arginine (Arg), l'acide aspartique (Asp), la leucine (Leu), la glycine (Gly), la sérine (Ser) et la proline (Pro).

L'extrait protéinique de guar peut ainsi comprendre :
- de 10 % à 30 % d'acide glutamique, notamment de 15 % à 25 %;
- de 5 % à 25 % d'arginine, notamment de 10 % à 20%, et plus particulièrement de 12 % à 16 %;
- de 5 % à 20 % d'acide aspartique, notamment de 10 % à 15 %;
- de 1 % à 10 % de leucine, et notamment de 5 à 10 % ;
- de 1 % à 8% de glycine, et notamment de 4 % à 6 % ;
les pourcentages étant exprimés en masse par rapport à la masse totale d'acides aminés contenu dans l'extrait.

De préférence, l'extrait protéinique de guar comprend les compositions en acides aminés suivantes :

| Acides aminés | % |
|---|---|
| cystéine | 1,38 |
| méthionine | 1,19 |
| acide aspartique | 10,90 |
| thréonine | 2,75 |
| Sérine | 4,83 |
| acide glutamique | 22,97 |
| Proline | 4,21 |
| Glycine | 5,19 |
| Alanine | 3,32 |
| Valine | 3,51 |
| isoleucine | 3,18 |
| Leucine | 6,21 |
| tyrosine | 3,70 |
| phénylalanine | 4,14 |
| Lysine | 3,78 |
| histidine | 2,89 |
| arginine | 14,41 |
| tryptophane | 1,44 |

les pourcentages étant exprimés en masse par rapport à la masse totale d'acides aminés de l'échantillon de la protéine isolée.

L'extrait protéinique de guar possède ainsi une quantité relativement importante d'arginine comparée aux protéines couramment utilisées dans le domaine cosmétique telles que les protéines de soja, les protéines de lait ou les protéines d'avoine. Or, l'arginine est un acide aminé particulièrement utile dans le domaine cosmétique car il possède par exemple une action hydratante sur la peau.

Sans vouloir se limiter à une quelconque théorie, les groupes chimiques sont susceptibles de se greffer notamment sur les fonctions -OH ou -NH₂ ou-COOH portées par les chaînes latérales des acides aminés et/ou les fonctions terminales des protéines.

De préférence, l'extrait protéinique comprend de 65 à 95% de protéines, et notamment de 65 à 85 % en poids de protéines.

L'extrait protéinique de guar sous forme d'un dérivé peut typiquement présenter la même répartition en acides aminés que l'extrait non dérivé, le cas échéant avec des masse molaires plus faibles.

Parmi les groupes chimiques que l'on peut greffer sur les acides aminés compris dans l'extrait protéinique, on peut citer:
- les groupes cationiques ou cationisables. Par "groupements cationisables", on entend des groupements qui sont potentiellement cationiques, i.e. qui peuvent être rendus cationiques en fonction du pH du milieu.

A noter que l'atome d'hydrogène n'est pas un groupe chimique au sens de la présente description.

Il est possible de combiner plusieurs modifications, par exemple une hydrolyse et un greffage. Il est possible de combiner des greffages de plusieurs groupes différents.

### Dérivés cationiques ou potentiellement cationiques

Parmi les groupements cationiques ou cationisables, on peut citer les groupements comprenant des ammoniums quaternaires ou des amines tertiaires, des pyridiniums, des guanidiniums, des phosphoniums ou des sulfoniums.

Les produits cationiques selon l'invention peuvent être obtenus en faisant réagir de façon classique les protéines de l'extrait protéinique de guar telles quelles ou après qu'elles aient subi une hydrolyse enzymatique ou chimique de manière à cliver les liaisons peptidiques.

### Cationisation par substitution nucléophile

L'introduction de groupements cationiques ou cationisables dans l'extrait protéinique de guar peut être réalisée par une réaction de substitution nucléophile.

Dans le cas où l'on souhaite introduire un groupement ammonium, le réactif adapté utilisé peut être :
- le chlorure de 3-chloro-2-hydroxypropyltriméthylammonium, vendu sous le nom de QUAB 188 par la société DEGUSSA ;
- un époxyde porteur d'un ammonium quaternaire tel que le chlorure de 2,3-époxypropyltriméthylammonium vendu sous le nom de QUAB 151 par la société DEGUSSA ou des composés analogues ;
- le chlorure de diéthylaminoéthyle ;
ou des accepteurs de Michaël comme, par exemple, des acrylates ou des méthacrylates porteurs d'ammonium quaternaires ou d'amines tertiaires.

### Cationisation par estérification

L'introduction de groupements cationiques ou cationisables sur les acides aminés de l'extrait de protéines de guar peut être réalisée par une estérification avec des acides aminés tels que, par exemple, la glycine, la lysine, l'arginine, l'acide 6-aminocaproïque ou avec des dérivés d'acides aminés quaternisés tel que, par exemple, le chlorhydrate de bétaïne.

### Cationisation par polymérisation radicalaire

L'introduction de groupements cationiques ou cationisables dans l'extrait protéinique de guar peut être réalisée par une polymérisation radicalaire comprenant le greffage de monomères comprenant au moins un groupement cationique ou cationisable sur les acides aminés de l'extrait protéique de guar.

L'amorçage radicalaire peut être effectué à l'aide de cérium comme cela est décrit dans la publication European Polymer Journal, vol. 12, p. 535-541, 1976. L'amorçage radicalaire peut également être effectué par un rayonnement ionisant et en particulier un bombardement sous faisceau d'électrons.

Les monomères comprenant au moins un groupement cationique ou cationisable mis en oeuvre pour réaliser cette polymérisation radicalaire peuvent être, par exemple, des monomères comprenant au moins une insaturation éthylénique et au moins un atome d'azote quaternaire ou quaternisable en ajustant le pH.

Parmi ces monomères comprenant au moins une insaturation éthylénique et au moins un atome d'azote quaternaire ou quaternisable en ajustant le pH, on peut citer les composés de formules (I), (II), (III), (IV) ou (V) suivants :
▪ le composé de formule générale (I) 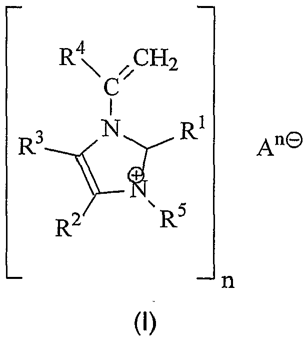 dans laquelle :
   - A^{nΘ} représente un ion Cl^{Θ} Br^{Θ}, I^{Θ} SO^{2Θ} CO₃^{2Θ} CH₃-OSO₃^{Θ}, OH^{Θ} ou CH₃-CH₂-OSO₃^{Θ},
   - R¹ à R⁵ identiques ou différents représentent, indépendamment les uns des autres, un groupe alkyle ayant de 1 à 20 atomes de carbone, un radical benzyle ou un atome de H, et

   n vaut 1 ou 2, ou
▪ le composé de formule générale (II) 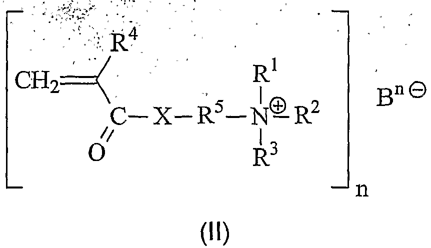 dans laquelle :
   - X représente un groupe -NH ou un atome d'oxygène O,
   - R⁴ représente un atome d'hydrogène ou un groupe alkyle ayant de 1 à 20 atomes de carbone,
   - R⁵ représente un groupe alcène ayant de 1 à 20 atomes de carbone,
   - R¹, R², R³ identiques ou différents représentent, indépendamment les uns des autres, un groupe alkyle ayant de 1 à 20 atomes de carbone,
   - Bn^{Θ} représente un ion Cl^{Θ} Br^{Θ}, I^{Θ}, SO₄^{2Θ} CO₃^{2Θ}, CH3-OSO₃^{Θ}, OH^{Θ} ou CH₃-CH₂-OSO₃^{Θ}, et
   - n vaut 1 ou 2, ou
▪ le composé de formule générale (III) 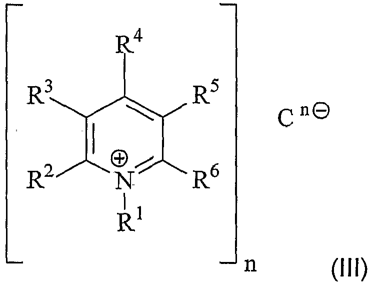 dans laquelle :
   - R¹ à R⁶ identiques ou différents représentent, indépendamment les uns des autres, un atome d'hydrogène ou un groupe alkyle ayant de 1 à 20 atomes de carbone, mais avec un des groupes R¹ à R⁶ représentant un groupe -CH = CH₂,
   - C^{nΘ} représente un ion Cl^{Θ}, Br^{Θ}, I^{Θ}, SO₄^{2Θ}, CO₃^{2Θ}, CHs-OSO₃^{Θ}, OH^{Θ} ou CH₃-CH₂-OSO₃^{Θ}, et
   - n vaut 1 ou 2, ou
▪ le composé de formule générale (IV) 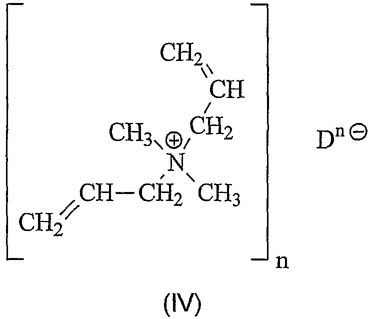 dans laquelle :
   - D^{nΘ} représente un ion Cl^{Θ} Br^{Θ}, I^{Θ}, SO₄^{2Θ}, CO₃^{2Θ}, CH₃-OSO₃^{Θ}, OH^{Θ} ou CH₃-CH₂-OSO₃^{Θ}, et
   - n vaut 1 ou 2.

De préférence, les monomères comprenant au moins une insaturation éthylénique et au moins un atome d'azote quaternaire ou quaternisable sont choisis parmi :
- l'acrylate de 2-diméthylaminoéthyle (ADAM),
- l'acrylate de 2-diméthylaminoéthyle quaternisé (ADAM-Quat),
- le méthacrylate de 2-diméthylaminoéthyle (MADAM),
- le méthacrylate de 2-diméthylaminoéthyle quaternisé (MADAM-Quat),
- le méthacrylate de 2-diéthylaminoéthyle quaternisé forme chlorure dénommé Pleximon 735 ou TMAE MC 80 par la société Röhm,
- le chlorure de diallyldiméthylammonium (DADMAC),
- le triméthyl ammonium propyl méthacrylamide forme chlorure dénommé MAPTAC, ou
- leurs mélanges.

L'extrait protéinique de guar dérivé cationique peut contenir des motifs cationiques ou cationisables issus d'une transformation chimique après polymérisation de monomères précurseurs de fonctions cationiques ou cationisables. On peut citer à titre d'exemple du poly-p-chlorométhylstyrène qui, après réaction avec une amine tertiaire telle qu'une triméthyl amine, forme du polyparatriméthylaminométhylstyrène quaternisé.

Les motifs cationiques ou cationisables sont associés avec des contre-ions chargés négativement. Ces contre-ions peuvent être choisis parmi les ions chlorures, bromures, iodures, fluorures, sulfates, méthylsulfates, phosphates, hydrogénophosphates, phosphonates, carbonates, hydrogénocarbonates, ou hydroxydes.

De préférence, on utilise des contre-ions choisis parmi les hydrogénophosphates, les méthylsulfates, les hydroxydes et les chlorures.

Le degré de substitution des extraits protéiniques de guar modifiés cationiques selon l'invention est d'au moins 0,01 et de préférence d'au moins 0,05.

Si le degré de substitution est inférieur à 0,01, l'efficacité de la fixation de l'extrait protéinique sur la surface à traiter peut être réduite.

Si le degré de substitution dépasse 0,05, l'efficacité en terme d'affinité pour la surface peut être nettement améliorée.

Le degré de substitution de l'extrait protéinique de guar modifié cationique correspond au nombre moyen de charges cationiques introduites par acide aminé. Ce degré de substitution peut être déterminé par analyse élémentaire, par exemple sur l'azote.

Selon un autre aspect, l'invention a pour objet un procédé de préparation d'un produit issu d'un extrait protéinique de guar, comprenant les étapes suivantes :
a) préparation d'un extrait protéinique de guar ;
b) réaction de greffons sur des fonctions d'acides aminés compris dans l'extrait protéinique ; et éventuellement
c) récupération du produit obtenu.

L'extrait protéinique de guar mis en oeuvre à l'étape a) peut être préparé à partir des graines de guar ou de préférence à partir de farine d'extraction de guar (guar meal), selon les techniques usuelles d'extraction des protéines à partir de végétaux, notamment de protéines de soja. De telles techniques sont décrites dans l'encyclopédie Kirk Othmer "Encyclopedia of Industrial Chemistry", vol. A22, pages 295 à 300 et pages 612 à 614.

L'extrait protéinique de guar peut notamment être isolé ou concentré à partir de la farine d'extraction de guar, laquelle est le sous-produit de la récupération des cupules des graines de guar. Cette farine d'extraction de guar est disponible dans le commerce. Elle est par exemple commercialisée par Rhodia sous le nom "Guar Meal" 100% ou 31%. Les farines d'extraction de guar peuvent être également préparées selon la méthode décrite par North J. P., Subramanian N., Narasinja Rao, M. S., J. Agric. Food Chem. 26 (5), 1243 (1978).

L'extrait protéinique de guar peut être préparée selon la méthode dite de « concentration ». Cette méthode comprend généralement :
a1) la mise en suspension de germes de guar, de préférence d'une farine d'extraction de guar, dans un liquide d'extraction ;
b1) la séparation de la phase solide S1 et la récupération de la phase liquide L1 ;
c1) l'ajustement du pH de la phase liquide L1 récupérée à un pH acide ;
d1) la récupération de l'extrait protéinique sous la forme d'un précipité.

Les germes de guar mis en oeuvre à l'étape a1) du procédé englobent les germes de guar qui peuvent être présents, par exemple dans les graines de guar éventuellement dépourvues de leur écorce et/ou broyées sous forme de poudre, dans les farines de guar ou encore dans les farines d'extraction de guar. De préférence, à l'étape a1), on procède à l'extraction de farines d'extraction de guar.

Le liquide d'extraction à l'étape a1) peut être choisi parmi les solvants organiques, de l'eau ou un mélange de ceux-ci.

Parmi les solvants organiques utiles à titre de liquide d'extraction, on peut par exemple citer les alcools tels que l'éthanol, les solvants hydrocarbonés tels que le n-hexane, les éthers tels que le diéthyl éther.

Le liquide d'extraction peut être également de l'eau, de préférence déminéralisée, et plus préférentiellement une solution à pH basique, éventuellement en combinaison avec un co-solvant organique, tel qu'un alcool.

Les solutions à pH basique sont des solutions de pH > 7, notamment > 8, et plus particulièrement > 9. Il peut s'agir notamment de solutions d'hydroxyde de métal alcalin telles que des solutions d'hydroxyde de sodium ou de potassium.

Le milieu d'extraction peut, en outre, contenir des sels minéraux tels que le chlorure de sodium ou de potassium.

La concentration des sels minéraux dans le milieu d'extraction peut varier dans une large mesure et est généralement comprise entre 0,5 M et 1,5 M.

L'extraction peut avoir lieu dans une vaste gamme de températures, notamment entre 20°C et 80°C, de préférence entre 40°C et 60°C, et plus préférentiellement à 55°C environ.

L'extraction peut être réalisée avec une proportion de farine d'extraction de guar:liquide d'extraction comprise entre 1:100 à 50 :100 exprimée en poids, de préférence avec une proportion comprise entre 2:100 et 25:100 exprimée en poids.

Le temps requis pour l'extraction peut également varier considérablement selon de nombreux facteurs, notamment la température de l'extraction et le liquide d'extraction. Une durée généralement comprise entre 10 mn et 3 heures s'avère généralement suffisante.

L'extrait brut obtenu à l'étape a1) est ensuite séparé par exemple par filtration ou centrifugation. La phase solide S1, moins riche en protéines et qui peut par exemple contenir de l'endosperme et/ou l'écorce, est éliminée, et la phase liquide L1, correspondant à l'extrait, riche en protéines, est récupérée.

Selon une variante, le solide S1 est récupéré à son tour et extrait avec un liquide d'extraction qui peut être identique ou différent du liquide d'extraction mis en oeuvre avec les germes de guar. L'extrait brut obtenu est ensuite séparé, et la phase liquide L2 est récupérée.

Les/la phase(s) liquide(s) extraite(s) L1 ou (L1 + L2) est/sont ensuite acidifiée(s) par addition d'une solution concentrée d'un acide minéral tel que l'acide chlorhydrique. Une quantité suffisante de cet acide est additionnée de façon à ce que le pH du liquide L1 ou L1+L2 soit ajusté à une valseur ≤ 7, en particulier ≤ 5, et plus particulièrement ≤ 4.

Le précipité qui se forme à la suite de cette opération est récupéré, par exemple par centrifugation ou filtration.

Il peut être ensuite séché, par exemple par concentration sous vide, atomisation ou lyophilisation. Il est à noter qu'une fraction particulière du précipité obtenu peut être purifiée ou concentrée par extraction liquide/liquide au moyen de solvants organiques ou par chromatographie préparative.

Selon une variante intéressante, l'extrait protéinique de guar est préparé à partir des farines d'extractions de guar ou de farines de guar selon la technique dite « d'isolation ». Cette méthode comprend les étapes mises en oeuvres dans l'étape de concentration à la différence que les germes de guar mis en oeuvre à l'étape a1) sous la forme de farines de guar ou d'extraction de guar sont préalablement enrichis en protéines selon les étapes de :
a2) tamisage des farines de guar ou d'extraction de guar et récupération des particules de diamètre inférieur à 1500 µm, notamment à 1400 µm ;
b2) séparation et récupération des particules les plus lourdes contenues dans la farine de guar ou d'extraction de guar tamisée.

Au cours de l'étape a2), une partie importante de l'endosperme de guar, pauvre en protéine, est éliminée.

L'étape b2) peut être réalisée selon des techniques conventionnelles, par exemple au moyen d'un sécheur à lit fluidisé, équipé d'un dispositif pour collecter les particules les plus légères entraînées par un courant d'air. Cette étape permet ainsi l'élimination des particules légères, riches en fibres, et la récupération des particules les plus denses qui sont généralement plus riches en protéines.

Les réactions des greffons sur les fonctions portées par les acides aminés contenus dans l'extrait protéinique de guar peuvent être réalisées par l'application ou l'adaptation de procédés de substitution nucléophile, d'estérification ou de polymérisations radicalaires utilisés jusque là ou décrits dans la littérature, par exemple ceux décrits dans R.C. Larock, Comprehensive Organic Transformations, VCH Publishers, 1989.

L'extrait protéinique obtenu peut être utilisé tel que résultant du procédé d'extraction, lequel peut conduire à une dépolymérisation partielle des protéines.

En variante, préalablement à l'étape de greffage b), le procédé peut comprendre en outre une réaction d'hydrolyse chimique ou enzymatique des liaisons peptidiques.

Selon une réalisation préférée, les protéines ont des masses moléculaires inférieures à 30 000 Da, notamment de 100-30 000, de préférence entre 500 et 20 000, et de façon plus préférentielle de l'ordre de 750 à 15 000 Da, lesquelles sont particulièrement préférées dans le domaine des produits cosmétiques. Les protéines hydrolysées sont en effet généralement plus substantives et pénètrent plus facilement dans le cortex du cheveu ou dans l'épiderme.

Parmi les enzymes utiles pour cette étape de dépolymérisation des protéines, on peut citer, par exemple, les protéases d'origine animale, végétales, microbiennes ou fongique.

Comme exemples de réactifs chimiques utiles pour cette étape de dépolymérisation, on peut citer les bases minérales telles que les hydroxydes de métal alcalin ou alcalino-terreux, les acides minéraux tels que l'acide chlorhydrique.

### Compositions - Utilisations

La composition pour la modification et/ou le traitement de surfaces comprend l'extrait protéinique de guar modifié, et généralement d'autres ingrédients (ou "composants"). En particulier elle comprend généralement un vecteur, le plus souvent liquide, appelé vecteur d'application topique pour les compositions cosmétiques ou pharmacologiques.

Ainsi des compositions utiles peuvent être des compositions pour le traitement et/ou la modification de surfaces comprenant:
- un vecteur liquide, par exemple un vecteur aqueux, alcoolique ou hydroxyalcoolique, etc
   l'extrait protéinique de guar modifié,
- éventuellement au moins un tensioactif, par exemple un tensioactif anionique, non ionique, amphotère, ou un mélange,
- éventuellement d'autres ingrédients.

La composition peut être utilisée dans un procédé de traitement ou de modification d'une surface, comprenant les étapes suivantes:
- appliquer sur la surface la composition, et
- éventuellement, éliminer le vecteur ou diluer la composition ou modifier le pH.

Les surfaces visées peuvent notamment être:
- la peau et/ou les cheveux pour les compositions cosmétiques
- les dents pour des compositions pour soins buccaux dentaires,
- les surfaces dures, dont la vaisselle, pour des compositions pour les soins ménagers, par exemple
   - les surfaces (métal, céramique, plastic, verre...) de la vaisselle, des formulations destinées au nettoyage de la vaisselle à la main ou en machine,
   - les sols (bois, céramique, plastic, béton...) pour des compositions de nettoyage multi-usage ou de nettoyage des sols,
   - les surfaces présentes dans les cuisines pour des compositions de nettoyage multi-usage ou de nettoyage des cuisines,
   - les surfaces présentes dans les salles de bain pour des compositions de nettoyage multi-usage ou de nettoyage des salles de bain,
   - les vitres ou pare-brise, pour des compositions de nettoyage des vitre ou des pare-brise
   - la peau pour des compositions pharmacologiques,
   - les feuilles des plantes pour des compositions phytosanitaires.
   - les surfaces textiles pour les compositions de nettoyage et/ou de rinçage (par exemple des assouplissants) et/ou de repassage du linge.

L'application d'une composition cosmétique selon l'invention est de préférence effectuée par voie topique.

La composition est destinée plus particulièrement au traitement de la peau ou du cheveu et peut se présenter sous forme d'onguent, de crème, d'huile, de lait, de pommade, de poudre, de tampon imbibé, de solution, de fluide, de gel, de spray, de lotion, de suspension, d'un produit moulé (un savon par exemple), de mousse. Les compositions cosmétiques selon l'invention peuvent ainsi se présenter sous la forme d'émulsion simple huile-dans-eau ou eau-dans-huile, d'émulsion multiple, de microémulsion, de gel aqueux ou hydroalcoolique.

Il peut notamment s'agir d'un shampooing, d'un après shampooing (rinçé ou non rinçé), d'un produit de coiffage (pour la mise en forme des cheveux par exemple) ou d'un gel douche.

Les compositions cosmétiques peuvent être des compositions pour le soin et l'hygiène de la peau et/ou du cheveu.

Des exemples de compositions cosmétiques pour cheveu, sont notamment des compositions de shampooing, d'après-shampooing, de produit de coiffage, de produits protecteurs, réparateurs, adoucissants ou encore de produits pour permanente et coloration.

Sans vouloir se limiter à une quelconque théorie, il a été observé que les extraits protéiniques de guar modifiés présentent une très bonne affinité pour le cheveu, ce qui pourrait expliquer leurs propriétés de fixation importantes, et notamment leur résistance à l'humidité sur cheveux secs.

Des exemples de compositions cosmétiques pour la peau sont notamment des produits pour le visage et le corps, des produits de jour ou de nuit, des produits solaires, des produits d'hygiène anti-âge ou anti-rides, des produits anti-pollution, des gels douches, des crèmes pour les mains.

Les compositions cosmétiques selon l'invention peuvent comprendre de 0,0001 à 4% dudit extrait par rapport au poids de la composition, de préférence de 0,01 à 1%.

Les protéines de guar contenues dans les compositions cosmétiques selon l'invention peuvent ainsi représenter de 0,00003% à 4 % en poids, notamment de 0,001% à 1 % en poids de la composition cosmétique.

Des exemples de compositions détergentes sont notamment des compositions à usage domestique et/ou de soins ménagers comme les produits pour le traitement des textiles tels que les lessives, les assouplissants, des produits d'entretien du linge, ou encore des produits de traitement des surfaces dures tels que les produits de nettoyage ou d'entretien des sols.

Les extraits protéiniques de guar modifiés selon l'invention sont particulièrement utiles pour le traitement des phanères, tels que le cheveu ou la peau ou encore des textiles ou des surfaces ménagères, ou bien encore des plantes, en particulier de la surface foliaire des plantes.

Les extraits protéiniques de guar modifiés peuvent être utilisés en association avec un véhicule cosmétiquement acceptable dans des compositions destinées à soigner et/ou réparer et/ou protéger la peau, les cheveux ou le cuir chevelu.

Les extraits protéiniques de guar modifiés peuvent ainsi être utilisés en association avec un véhicule cosmétiquement acceptable, pour augmenter ou améliorer l'hydratation, l'élasticité, l'aspect satiné de la peau, ou encore pour raffermir la peau.

Ils peuvent également être utilisés en association avec un véhicule cosmétiquement acceptable, dans des compositions antipelliculaires, repousse du cheveu, antichute du cheveu, des compositions de soin du cheveu hydratantes, nourrissantes, revitalisantes. Ils peuvent être utilisés dans des compositions destinées à la protection du cheveu contre les agressions dues au froid, au soleil ou à la pollution (compositions dites « winter care », « summer care », anti-pollution). Enfin, ils peuvent être utilisés dans des compositions destinées à apporter du volume, de la brillance, de l'éclat à la couleur naturelle ou à la coloration, un toucher agréable, du ressort aux boucles, un effet lissant.

Par "véhicule cosmétiquement acceptable", on entend un véhicule adapté pour une utilisation en contact avec des cellules humaines et animales, en particulier des cellules de l'épiderme, sans toxicité, irritation, réponse allergique indue et similaire, et proportionné à un rapport avantage / risque raisonnable.

Les extraits protéiniques de guar modifiés peuvent être utilisés dans les lessives, les assouplissants, les produits d'entretien pour la maison, par exemple les produits de nettoyage pour les sols ou les surfaces domestiques, et les produits anti-poussière.

De façon avantageuse, les produits selon l'invention présentent un effet adoucissant et anti-froissage s'agissant du traitement des textiles, ou encore un effet anti-traces, anti-salissures s'agissant des surfaces ménagères.

Sans vouloir se limiter à une quelconque théorie, l'application des produits selon l'invention conduirait à hydrophiliser les surfaces textiles ou ménagères, ce qui permettrait d'empêcher la formation de traces au séchage et de faciliter le nettoyage suivant.

Les extraits protéiniques de guar modifiés peuvent être utilisés dans une composition phytosanitaire et/ou d'éléments nutritionnels destinée à être pulvérisée sur la surface foliaire des plantes, en tant qu'agent anti-rebond. Cet agent anti-rebond permet avantageusement d'améliorer l'adhésion instantanée et, par conséquent, la rétention et donc l'efficacité de la composition pulvérisée.

Des exemples de compositions phytosanitaires sont des formulations contenant une matière active telle qu'un herbicide, défanant, débroussaillant, bactéricide, fongicide, insecticide, acaricide, régulateur de croissance.

Ainsi, les extraits protéiniques de guar modifiés permettent de limiter la perte au sol des compositions pulvérisées, laquelle est susceptible de générer une pollution des sols et des nappes phréatiques souterraines.

Pour être plus complet, les produits selon l'invention ou les extraits protéiniques de guar modifiés selon l'invention peuvent être employés dans :
- des compositions cosmétiques,
- des compositions pharmacologiques,
- des compositions pour les soins ménagers,
- des compostions de revêtement telles que des peintures,
- des compositions alimentaires pour l'alimentation des humains,
- des compositions de lubrification,
- des fluides mis en oeuvres dans l'exploitation des gisements de pétrole ou de gaz,
- des compositions phytosanitaires,
- les traitements de l'eau.

Les produits selon l'invention ou les extraits protéiniques de guar modifiés selon l'invention peuvent être employés à titre d'agent:
- de traitement et/ou modification des surfaces,
- agent dispersant,
- agent émulsifiant,
- agent anti-rebond, agent anti-lessivation, agent anti-dérive, de formulations phytosanitaires,
- agent d'adhésion,
- agent floculant,
- agent tensioactif,
- agent de modulation des propriétés de mousses,
- agent de stabilisation de mousses
- agent de modification de l'apparence d'un revêtement, et/ou
- agent viscosant.

Dans les compositions, l'extrait protéinique de guar, le cas échéant sous forme d'un dérivé, peut avoir un effet de stabilisant de mousse, notamment dans des compositions cosmétiques moussantes, ou dans des compositions de nettoyage de la vaisselle ou du linge à la main.

D'autres détails ou avantages pourront apparaître au vu des exemples qui suivent.

### Exemple 1: Préparation d'un extrait protéinique de guar "protéine de guar"

On prépare une suspension contenant 10 % de guar meal en dispersant 45,3 kg de guar meal (Rhodia, usine de Vernon) dans 454 L d'eau.préchauffée à 55°C. Le pH initial de la suspension est de 4,85. On ajoute 1980 mL de soude 30 % pour relever le pH à 9,53. La suspension est maintenue 45 minutes à 55°C sous agitation.

La suspension est centrifugée, et on récupère 394,4 kg de liquide (1) et 107,7 kg de solide (2). Le solide (2) n'est pas réutilisé.

Le pH du liquide (1) est de 8,62. On ajoute 3920 mL d'acide chlorhydrique 30% pour abaisser le pH à 4,54, ce qui entraîne la précipitation des protéines. Cette suspension est maintenue 30 minutes à 45°C sous agitation.

La suspension est centrifugée. Le solide (3) est récupéré et on élimine 310,5 kg de liquide (4).

Le solide (3) est à nouveau suspendu dans de l'eau pour être lavé. On ajoute une quantité d'eau environ identique à la masse de solide (3). Le pH de cette suspension est de 4,75.

Cette suspension est centrifugée. On récupère 46,3 kg de solide (4) et 110,7 kg de liquide (5). Le liquide (5) n'est pas réutilisé.

On ajoute 510 mL de soude 30% au solide humide (4) pour amener le pH à 6,94.

Ce solide est ensuite pasteurisé par traitement thermique à 90°C pendant 20 secondes, puis atomisé. On obtient ainsi 6,8 kg de protéines de guar isolées.

L'échantillon de la protéine isolée contient :
71-72% protéines
5,6% cendres (calcination)
6,6% matières grasses (hydrolyse / extraction)
8,8% teneur en eau (Karl Fischer)]

### Sucres (HPLC/Réfractométrie)

Fructose <0,1%
Glucose <0,1%
Saccharose 0,3%
Maltose <0,5%
Lactose <0,5%

### Aminogramme

Acide aminé % en masse sur le contenu total en acides aminés de l'échantillon de la protéine isolée.

| *Acides aminés* | % |
|---|---|
| cystéine | 1,38 |
| méthionine | 1,19 |
| acide aspartique | 10,90 |
| thréonine | 2,75 |
| serine | 4,83 |
| acide glutamique | 22,97 |
| proline | 4,21 |
| glycine | 5,19 |
| alanine | 3,32 |
| valine | 3,51 |
| isoleucine | 3,18 |
| leucine | 6,21 |
| tyrosine | 3,70 |
| phénylalanine | 4,14 |
| lysine | 3,78 |
| histidine | 2,89 |
| arginine | 14,41 |
| tryptophan | 1,44 |

Acide aminé % en masse sur l'échantillon entier de protéine isolée.

| | |
|---|---|
| cystéine | 0,98 |
| méthionine | 0,84 |
| acide aspartique | 7,72 |
| thréonine | 1,95 |
| serine | 3,42 |
| acide glutamique | 16,27 |
| proline | 2,98 |
| glycine | 3,68 |
| alanine | 2,35 |
| valine | 2,49 |
| isoleucine | 2,25 |
| leucine | 4,4 |
| tyrosine | 2,62 |
| phénylalanine | 2,93 |
| lysine | 2,68 |
| histidine | 2,05 |
| arginine | 10,21 |
| tryptophane | 1,02 |

Masse moléculaire de la protéine : 13133 Da (Analyse MALDI-TOF-MS). Métaux lourds : As+Cd+Cr+ Ni+Hg+Pb+Se+Sn <15 ppm.

### Exemple 2 : Préparation d'un extrait protéinique de guar dérivé, cationisé

Modification d'un extrait protéinique de guar pour introduire des groupements cationiques de type triméthylammonium. On utilise comme composé de départ l'extrait protéinique de guar de l'exemple 1.

Dans un réacteur en verre double-enveloppe de 1. litre équipé d'une agitation mécanique et d'un réfrigérant ascendant, on introduit 160 ml d'eau déminéralisée, puis 0,75 g d'hydroxyde de sodium en pastilles. L'agitation est mise en marche à 50 tours par minutes pour dissoudre la soude solide. Une fois l'hydroxyde de sodium dissout, on ajoute 30 g de poudre d'extrait protéinique de guar présentant un taux d'humidité de 7,3% en masse.

Le réacteur est porté à 60°C masse au moyen d'une circulation de fluide caloporteur chaud dans la double enveloppe. Après 1 heure à 60°C sous agitation, on ajoute en goutte à goutte sur 20 minutes un volume de 66 ml de Quab® 151 (solution de chlorure de 2,3-époxypropyltriméthylammonium à 70% en masse dans l'eau, commercialisée par la société Degussa). Après addition, le mélange réactionnel est maintenu sous agitation à 60°C pendant 5 heures.

Après refroidissement et retour à température ambiante, on ajoute au milieu réactionnel de l'acide acétique glacial jusqu'à atteindre un pH égal à 7.

Le contenu du réacteur est transvasé dans une ampoule à décanter et additionné en goutte à goutte à 2 litres d'éthanol absolu agricole placés sous agitation. Un précipité apparaît. Ce solide est lavé par une succession de 3 séquences d'opérations de décantation, élimination du surnageant, remise en suspension dans 1,5 litre d'éthanol frais. En final, le solide est essoré sur entonnoir filtrant en verre fritté de porosité 2. Il est séché pendant 16 heures à 45°C sous vide 200 mbar compensé à 180 mbar avec de l'azote. On obtient en final 21,6 g de solide en poudre.

### Propriétés de la protéine de guar cationisée

### Point isoélectrique de l'extrait protéinique cationisée (absence de sel)

Le point isoélectrique d'extrait protéinique cationisé de l'exemple 1 a été déterminé en mesurant la transmittance de la solution au moyen d'un spectrophotomètre UV-Vis à 600 nm, en fonction du pH mesuré par un pHmètre.

On réalise un diagramme qui montre l'influence du pH sur la turbidité et donc la solubilité d'une solution à 0,5% en protéine de guar cationisée dans l'eau déminéralisée.

Aucune précipitation n'est observée mais à un pH de 11,4 la solution devient très turbide et la transmittance est alors nulle. Puis, en augmentant encore le pH, la solution devient plus claire. Le point isoélectrique de la protéine cationisée se trouve déplacé aux pH plus élevés (à pH =11,4 environ) du fait de la cationisation.

Le point isoélectrique de l'extrait protéinique cationisé se situe donc aux environs de pH = 11,4.

### Exemple 3 : Shampooings et formulabilité

La formulation classique utilisée comprend les composants suivants :
- 0,3% d'extrait protéinique de guar cationisé ;
- 2% de tensioactif amphotère ;
- 14% de tensioactif anionique ;
- 1-2% de sel NaCl ;
- de l'eau jusqu'à atteindre 100% de formulation.

Les tensioactifs utilisés :
CAPB : cocamidopropyl bétaine (tensioactif amphotère) ;
SLES : lauryléthersulfate de sodium (tensioactif anionique).

### Mode opératoire

Le mode opératoire pour obtenir une formulation de shampooing appropriée est le suivant :
- mélanger la protéine dans l'eau dans un bécher, agiter jusqu'à dissolution (durée très variable suivant le polymère, peut nécessiter une modification du pH) ;
- ajouter le sel, agiter jusqu'à dissolution ;
- pendant ce temps, mélanger les deux tensioactifs dans un autre bêcher pendant 30 minutes ;
- verser l'eau qui contient le sel et le polymère dans le bêcher contenant les tensioactifs. Agiter 2h ;
- ajuster le pH entre 5,5 et 6,5 avec de la soude ou de l'acide citrique.

## Revendications

1. Produit issu d'un extrait protéinique de guar, **caractérisé en ce qu'**il comprend des groupes greffés sur des fonctions d'acides aminés compris dans l'extrait protéinique, lesdits groupes greffés comprenant des groupes cationiques ou cationisables choisis parmi des groupements comprenant des ammoniums quaternaires ou des amines tertiaires, des pyridiniums, des guanidiniums, des phosphoniums ou des sulfoniums.

2. Produit selon la revendication 1, **caractérisé en ce que** l'extrait protéinique comprend au moins 65 % en poids de protéines.

3. Produit selon la revendication 1 ou 2, **caractérisé en ce que** l'introduction de groupements cationiques ou cationisables dans l'extrait protéinique de guar est réalisée par une réaction de substitution nucléophile.

4. Produit selon la revendication 1 ou 2, **caractérisé en ce que** l'introduction de groupes cationiques ou cationisables dans l'extrait protéinique de guar est réalisée par une estérification avec des acides aminés tel que par exemple la glycine, la lysine, l'arginine, l'acide 6-aminocaproïque, ou avec des dérivés d'acides aminés quaternisés tel que, par exemple, le chlorhydrate de bétaïne.

5. Produit selon la revendication 1 ou 2, **caractérisé en ce que** l'introduction de groupes cationiques ou cationisables dans l'extrait protéinique de guar est réalisée par une polymérisation radicalaire comprenant le greffage de monomères comprenant au moins un groupement cationique ou cationisable sur les acides aminés de l'extrait protéique de guar.

6. Produit selon la revendication 5, **caractérisé en ce que** les monomères comprenant au moins un groupe cationique ou cationisable mis en oeuvre pour réaliser cette polymérisation radicalaire sont choisis parmi les composés de formules (I), (II), (III) ou (IV) suivants :
▪ le composé de formule générale (I) 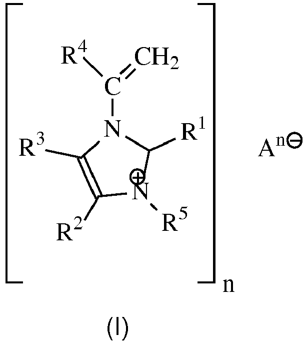 dans laquelle :
- A^{nΘ} représente un ion Cl^{Θ}, Br^{Θ}, I^{Θ}, SO₄^{2Θ}, CO₃^{2Θ}, CH₃-OSO₃^{Θ} ,OH^{Θ} ou CH₃-CH₂-OSO₃^{Θ},
- R¹ à R⁵ identiques ou différents représentent, indépendamment les uns des autres, un groupe alkyle ayant de 1 à 20 atomes de carbone, un radical benzyle ou un atome de H, et
n vaut 1 ou 2, ou
▪ le composé de formule générale (II) 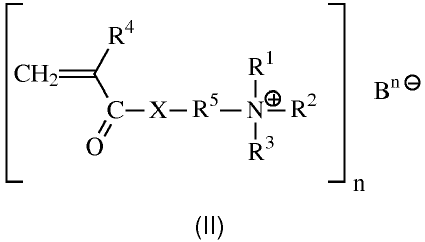 dans laquelle :
- X représente un groupe -NH ou un atome d'oxygène O,
- R⁴ représente un atome d'hydrogène ou un groupe alkyle ayant de 1 à 20 atomes de carbone,
- R⁵ représente un groupe alcène ayant de 1 à 20 atomes de carbone,
- R¹, R², R³ identiques ou différents représentent, indépendamment les uns des autres, un groupe alkyle ayant de 1 à 20 atomes de carbone,
- B^{nΘ} représente un ion Cl^{Θ}, Br^{Θ}, I^{Θ}, SO₄^{2Θ}, CO₃^{2Θ}, CH3-OSO₃^{Θ}, OH^{Θ} ou CH₃-CH₂-OSO₃^{Θ}, et
- n vaut 1 ou 2, ou
▪ le composé de formule générale (III) 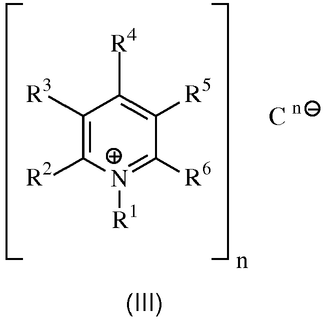 dans laquelle :
- R¹ à R⁶ identiques ou différents représentent, indépendamment les uns des autres, un atome d'hydrogène ou un groupe alkyle ayant de 1 à 20 atomes de carbone, mais avec un des groupes R¹ à R⁶ représentant un groupe -CH = CH₂ ,
- C^{nΘ} représente un ion Cl^{Θ}, Br^{Θ}, I^{Θ}, SO₄^{2Θ}, CO₃^{2Θ}, CH3-OSO₃^{Θ}, OH^{Θ} ou CH₃-CH₂-OSO₃^{Θ}, et
- n vaut 1 ou 2, ou
▪ le composé de formule générale (IV) 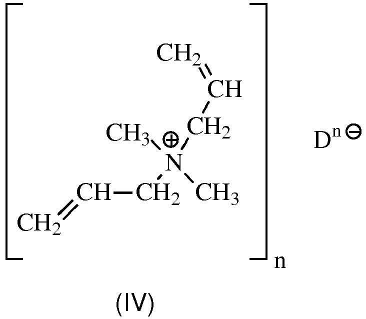 dans laquelle :
- D^{nΘ} représente un ion Cl^{Θ}, Br^{Θ}, I^{Θ}, SO₄^{2Θ}, CO₃^{2Θ}, CH3-OSO₃^{Θ}, OH^{Θ} ou CH₃-CH₂-OSO₃^{Θ}, et
- n vaut 1 ou 2.

7. Produit selon la revendication 5 ou 6, **caractérisé en ce que** les monomères comprenant au moins un groupe cationique ou cationisable mis en oeuvre pour réaliser cette polymérisation radicalaire sont choisis parmi :
- l'acrylate de 2-diméthylaminoéthyle (ADAM),
- l'acrylate de 2-diméthylaminoéthyle quaternisé (ADAM-Quat),
- le méthacrylate de 2-diméthylaminoéthyle (MADAM),
- le méthacrylate de 2-diméthylaminoéthyle quaternisé (MADAM-Quat)
- le méthacrylate de 2-diéthylaminoéthyle quaternisé forme chlorure dénommé Pleximon 735 ou TMAE MC 80 par la société Röhm,
- le chlorure de diallyldiméthylammonium (DADMAC),
- le triméthyl ammonium propyl méthacrylamide forme chlorure dénommé MAPTAC ou
- leurs mélanges.

8. Produit selon l'une des revendications 1 à 7, **caractérisé en ce que** les groupes cationiques ou cationisables sont associés avec des contre-ions chargés négativement choisis parmi les ions chlorures, bromures, iodures, fluorures, sulfates, méthylsulfates, phosphates, hydrogénophosphates, phosphonates, carbonates, hydrogénocarbonates, ou hydroxydes.

9. Produit selon l'une quelconque des revendications précédentes, dans laquelle l'extrait protéinique comprend de 65 à 95 % en poids de protéines.

10. Produit selon la revendication 9, dans laquelle l'extrait protéinique comprend de 65 à 85 % en poids de protéines.

11. Produit selon l'une quelconque des revendications précédentes, dans laquelle l'extrait protéinique comprend :
- de 10 % à 30 % d'acide glutamique ;
- de 5 % à 25 % d'arginine,
- de 5 % à 20 % d'acide aspartique ;
- de 1 % à 10 % de leucine ;
- de 1 % à 8% de glycine ;
les pourcentages étant exprimés en masse par rapport à la masse totale d'acides aminés contenus dans l'extrait.

12. Procédé de préparation d'un produit issu d'extrait protéinique de guar, selon l'une quelconque des revendications précédentes, comprenant les étapes suivantes:
a) préparation d'un extrait protéinique de guar ;
b) réaction de greffons sur des fonctions d'acides aminés compris dans l'extrait protéinique ; et éventuellement
c) récupération du produit obtenu.

13. Utilisation du produit selon l'une quelconque des revendications 1 à 11 dans:
- des compositions cosmétiques,
- des compositions pour les soins ménagers,
- des compostions de revêtement telles que des peintures,
- des compositions alimentaires pour l'alimentation des humains,
- des compositions de lubrification,
- des fluides mis en oeuvres dans l'exploitation des gisements de pétrole ou de gaz,
- des compositions phytosanitaires,
- les traitements de l'eau.

14. Utilisation selon la revendication précédente à titre d'agent:
- de traitement et/ou modification des surfaces,
- agent dispersant,
- agent émulsifiant,
- agent anti-rebond, agent anti-lessivation, agent anti-dérive, de formulations phytosanitaires,
- agent d'adhésion,
- agent floculant,
- agent tensioactif,
- agent de modulation des propriétés de mousses,
- agent de stabilisation de mousses,
- agent de modification de l'apparence d'un revêtement, et/ou
- agent viscosant.

15. Composition pharmacologique comprenant le produit selon l'une quelconque des revendications 1 à 11.

## Patentansprüche

1. Produkt aus einem Guar-Proteinextrakt, **dadurch gekennzeichnet, dass** er auf die Funktionen von in dem Extrakt vorhandenen Aminosäuren gepfropfte Gruppen umfasst, wobei die gepfropften Gruppen kationische oder kationisierbare Gruppen umfassen, die aus Gruppen ausgewählt sind, die quaternäre Ammoniumgruppen oder tertiäre Amine, Pyridiniumgruppen, Guanidiniumgruppen, Phosphoniumgruppen oder Sulfoniumgruppen umfassen.

2. Produkt nach Anspruch 1, **dadurch gekennzeichnet, dass** der Proteinextrakt mindestens 65 Gew.-% Proteine umfasst.

3. Produkt nach Anspruch 1 oder 2, **dadurch gekennzeichnet, dass** die Einführung von kationischen oder kationisierbaren Gruppen in dem Guar-Proteinextrakt durch eine nukleophile Substitutionsreaktion durchgeführt wird.

4. Produkt nach Anspruch 1 oder 2, **dadurch gekennzeichnet, dass** die Einführung von kationischen oder kationisierbaren Gruppen in dem Guar-Proteinextrakt durch eine Veresterung mit Aminosäuren, wie zum Beispiel Glycin, Lysin, Arginin, 6-Aminocapronsäure, oder mit quaternisierten Aminosäurederivaten, wie zum Beispiel Betainhydrochlorid, durchgeführt wird.

5. Produkt nach Anspruch 1 oder 2, **dadurch gekennzeichnet, dass** die Einführung von kationischen oder kationisierbaren Gruppen in dem Guar-Proteinextrakt durch eine radikalische Polymerisation durchgeführt wird, umfassend das Pfropfen von Monomeren, die mindestens eine kationische oder kationisierbare Gruppe umfassen, auf die Aminosäuren des Guar-Proteinextrakts.

6. Produkt nach Anspruch 5, **dadurch gekennzeichnet, dass** die zur Durchführung dieser radikalischen Polymerisation eingesetzten Monomere, die mindestens eine kationische oder kationisierbare Gruppe umfassen, aus den folgenden Verbindungen der Formeln (I), (II), (III) oder (IV) ausgewählt sind:
▪ der Verbindung der allgemeinen Formel (I) 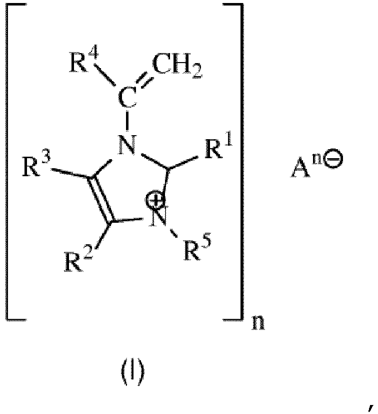 worin:
- A^{n⊝} für ein Ion Cl^{⊝} Br^{⊝}, I^{⊝}, SO₄^{2⊝}, CO₃^{2⊝}, CH₃-OSO₃^{⊝}, OH^{⊝} oder CH₃-CH₂-OSO₃^{⊝} steht,
- R¹ bis R⁵, die gleich oder verschieden sind, unabhängig voneinander eine Alkylgruppe mit 1 bis 20 Kohlenstoffatomen, einen Benzylrest oder ein H-Atom darstellen und
n einen Wert von 1 oder 2 hat, oder
▪ der Verbindung der allgemeinen Formel (II) 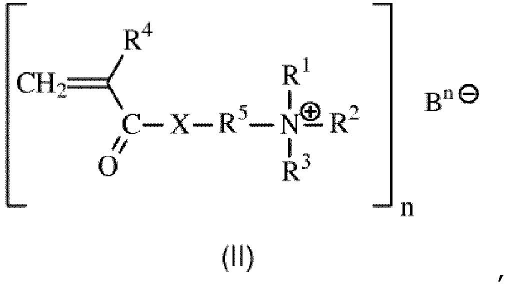 worin:
- X für eine Gruppe -NH oder für ein Sauerstoffatom 0 steht,
- R⁴ für ein Wasserstoffatom oder eine Alkylgruppe mit 1 bis 20 Kohlenstoffatomen steht,
- R⁵ für eine Alkengruppe mit 1 bis 20 Kohlenstoffatomen steht,
- R¹, R², R³, die gleich oder verschieden sind, unabhängig voneinander für eine Alkylgruppe mit 1 bis 20 Kohlenstoffatomen stehen,
- B^{n⊝} für ein Ion Cl^{⊝}, Br^{⊝}, I^{⊝}, SO₄^{2⊝} CO₃^{2⊝} CH₃-OSO₃^{⊝}, OH^{⊝} oder CH₃-CH₂-OSO₃^{⊝} steht und
n einen Wert von 1 oder 2 hat, oder
▪ der Verbindung der allgemeinen Formel (III) 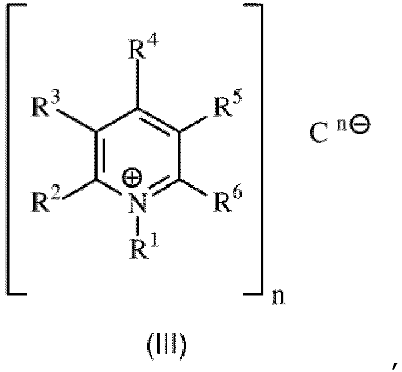 worin:
- R¹ bis R⁶, die gleich oder verschieden sind, unabhängig voneinander für ein Wasserstoffatom oder eine Alkylgruppe mit 1 bis 20 Kohlenstoffatomen stehen, wobei aber eine der Gruppen R¹ bis R⁶ für eine Gruppe-CH=CH₂ steht,
- C^{n⊝} für ein Ion Cl^{⊝}, Br^{⊝}, I^{⊝}, SO₄^{2⊝} CO₃^{2⊝}, CH₃-OSO₃^{⊝}, OH^{⊝} oder CH₃-CH₂-OSO₃^{⊝} steht und
n einen Wert von 1 oder 2 hat, oder
▪ der Verbindung der allgemeinen Formel (IV) 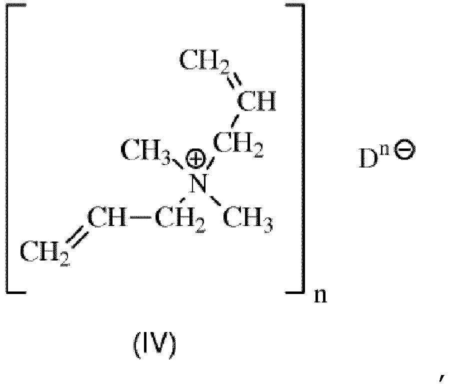 worin:
- D^{n⊝} für ein Ion Cl^{⊝} Br^{⊝}, I^{⊝}, SO₄^{2⊝} CO₃^{2⊝} CH₃-OSO₃^{⊝}, OH^{⊝} oder CH₃-CH₂-OSO₃^{⊝} steht und
n einen Wert von 1 oder 2 hat.

7. Produkt nach Anspruch 5 oder 6, **dadurch gekennzeichnet, dass** die für die Durchführung dieser radikalischen Polymerisation eingesetzten Monomere, die mindestens eine kationische oder kationisierbare Gruppe umfassen, aus den Folgenden ausgewählt sind:
- 2-Dimethylaminoethylacrylat (ADAM),
- quaternisiertem 2-Dimethylaminoethylacrylat (ADAM-Quat),
- 2-Dimethylaminoethylmethacrylat (MADAM),
- quaternisiertem 2-Dimethylaminoethylmethacrylat (MADAM-Quat),
- quaternisiertem 2-Diethylaminoethylmethacrylat in Chloridform, als Pleximon 735 oder TMAE MC 80 bezeichnet, von der Firma Röhm,
- dem Chlorid von Diallyldimethylammoniumchlorid (DADMAC),
- Trimethylammoniumpropylmethacrylamid in Chloridform, als MAPTAC bezeichnet, oder
- deren Gemische.

8. Produkt nach einem der Ansprüche 1 bis 7, **dadurch gekennzeichnet, dass** die kationischen oder kationisierbaren Gruppen mit negativ geladenen Gegenionen assoziiert sind, die aus Chlorid-, Bromid-, Iodid-, Fluorid-, Sulfat-, Methylsulfat-, Phosphat-, Hydrogenphosphat-, Phosphonat-, Carbonat-, Hydrogencarbonat- oder Hydroxidionen ausgewählt sind.

9. Produkt nach einem der vorhergehenden Ansprüche, wobei der Proteinextrakt 65 bis 95 Gew.-% Proteine umfasst.

10. Produkt nach Anspruch 9, wobei der Proteinextrakt 65 bis 85 Gew.-% Proteine umfasst.

11. Produkt nach einem der vorhergehenden Ansprüche, wobei der Proteinextrakt Folgendes umfasst:
- 10% bis 30% Glutaminsäure,
- 5% bis 25% Arginin,
- 5% bis 20% der Asparaginsäure,
- 1% bis 10% Leucin,
- 1% bis 8% Glycin,
wobei die Prozentsätze in Massenprozent, bezogen auf die Gesamtmasse an in dem Extrakt enthaltenen Aminosäuren ausgedrückt sind.

12. Verfahren zur Herstellung eines Produkts aus Guar-Proteinextrakt nach einem der vorhergehenden Ansprüche, umfassend die folgenden Schritte:
a) Herstellung eines Guar-Proteinextrakts,
b) Pfropfungsreaktion an den Funktionen von im Proteinextrakt enthaltenen Aminosäuren und gegebenenfalls
c) Gewinnen des erhaltenen Produkts.

13. Verwendung des Produkts nach einem der Ansprüche 1 bis 11 in:
- Kosmetikzusammensetzungen,
- Haushaltszusammensetzungen,
- Beschichtungszusammensetzungen, wie Anstrichfarben,
- Nahrungsmittelzusammensetzungen für die Ernährung von Menschen,
- Schmierungszusammensetzungen,
- bei der Ausbeutung von Öl- oder Gasvorkommen eingesetzten Fluiden,
- phytosanitären Zusammensetzungen,
- Wasserbehandlungen.

14. Verwendung nach dem vorhergehenden Anspruch als Mittel:
- zur Behandlung und/oder Modifikation von Oberflächen,
- Dispergiermittel,
- Emulgator,
- Anti-Abprall-Mittel, Anti-Lessivierungs-Mittel, Anti-Drift-Mittel von Pflanzenschutzformulierungen,
- Adhäsionsmittel,
- Flockungsmittel,
- oberflächenaktives Mittel,
- Mittel zum Modulieren der Eigenschaften von Schäumen,
- Schaumstabilisierungsmittel,
- Mittel zur Modifikation des Aussehens einer Beschichtung und/oder
- Verdickungsmittel.

15. Pharmakologische Zusammensetzung, umfassend das Produkt nach einem der Ansprüche 1 bis 11.

## Claims

1. Product derived from a guar protein extract, **characterized in that** it comprises groups grafted onto functions of amino acids contained in the protein extract, said grafted groups comprising cationic or cationizable groups selected from groups comprising quaternary ammoniums or tertiary amines, pyridiniums, guanidiniums, phosphoniums or sulphoniums.

2. Product according to Claim 1, **characterized in that** the protein extract comprises at least 65% by weight of proteins.

3. Product according to Claim 1 or 2, **characterized in that** the introduction of cationic or cationizable groups into the guar protein extract is carried out by a nucleophilic substitution reaction.

4. Product according to Claim 1 or 2, **characterized in that** the introduction of cationic or cationizable groups into the guar protein extract is carried out by esterification with amino acids such as, for example, glycine, lysine, arginine or 6-aminocaproic acid, or with quaternized amino acid derivatives such as, for example, betaine hydrochloride.

5. Product according to Claim 1 or 2, **characterized in that** the introduction of cationic or cationizable groups into the guar protein extract is carried out by radical polymerization comprising the grafting of monomers, comprising at least one cationic or cationizable group, onto the amino acids of the guar protein extract.

6. Product according to Claim 5, **characterized in that** the monomers comprising at least one cationic or cationizable group used for carrying out this radical polymerization are selected from the compounds of the following formula (I), (II), (III) or (IV):
• the compound of general formula (I) 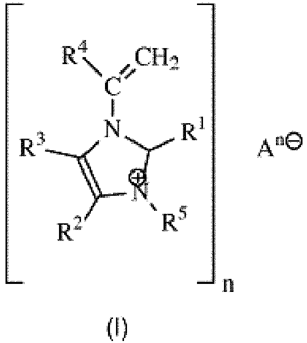 wherein:
- Aⁿ⁻ represents a Cl⁻, Br⁻, I⁻, SO₄²⁻, CO₃²⁻, CH₃-OSO₃⁻, OH⁻, or CH₃-CH₂-OSO₃⁻ ion,
- R¹ to R⁵, which are identical or different, represent, independently of one another, an alkyl group having from 1 to 20 carbon atoms, a benzyl radical or an H atom, and
- n is equal to 1 or 2, or
• the compound of general formula (II) 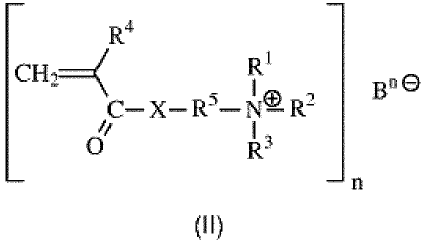 wherein:
- X represents an -NH group or an atom of oxygen 0,
- R⁴ represents a hydrogen atom or an alkyl group having from 1 to 20 carbon atoms,
- R⁵ represents an alkene group having from 1 to 20 carbon atoms,
- R¹, R², and R³, which are identical or different, represent, independently of one another, an alkyl group having from 1 to 20 carbon atoms,
- Bⁿ⁻ represents a Cl⁻, Br⁻, I⁻, SO₄²⁻, CO₃²⁻, CH₃-OSO₃⁻, OH⁻, or CH₃-CH₂-OSO₃⁻ ion, and
- n is equal to 1 or 2, or
• the compound of general formula (III) 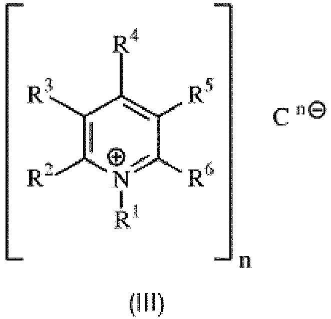 wherein:
- R¹ to R⁶, which are identical or different, represent, independently of one another, a hydrogen atom or an alkyl group having from 1 to 20 carbon atoms, but with one of the groups R¹ to R⁶ representing a -CH=CH₂ group,
- Cⁿ⁻ represents a Cl⁻, Br⁻, I⁻, SO₄²⁻ , CO₃²⁻, CH-OSO₃⁻ , OH⁻, or CH₃-CH₂-OSO₃⁻ ion, and
- n is equal to 1 or 2, or
• the compound of general formula (IV) 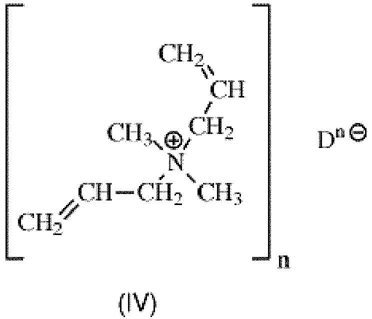 wherein:
- Dⁿ represents a Cl⁻, Br⁻, I⁻, SO₄²⁻ , CO₃²⁻, CH₃-OSO₃⁻, OH⁻, or CH₃-CH₂-OSO₃- ion, and
- n is equal to 1 or 2.

7. Product according to Claim 5 or 6, **characterized in that** the monomers comprising at least one cationic or cationizable group used to carry out this radical polymerization are selected from:
- 2-dimethylaminoethyl acrylate (ADAM),
- quaternized 2-dimethylaminoethyl acrylate (ADAM-quat),
- 2-dimethylaminoethyl methacrylate (MADAM),
- quaternized 2-dimethylaminoethyl methacrylate (MADAM-quat),
- quaternized 2-dimethylaminoethyl methacrylate, chloride form, referred to as Pleximon 735 or TMAE MC 80 by Röhm,
- diallyldimethylammonium chloride (DADMAC),
- methacrylamidopropyltrimethylammonium, chloride form, referred to as MAPTAC, or
- mixtures thereof.

8. Product according to one of Claims 1 to 7, **characterized in that** the cationic or cationizable groups are associated with negatively charged counterions selected from chloride, bromide, iodide, fluoride, sulphate, methylsulphate, phosphate, hydrogen phosphate, phosphonate, carbonate, hydrogen carbonate or hydroxide ions.

9. Product according to any one of the preceding claims, wherein the protein extract comprises from 65% to 95% by weight of proteins.

10. Product according to Claim 9, wherein the protein extract comprises from 65% to 85% by weight of proteins.

11. Product according to any one of the preceding claims, wherein the protein extract comprises:
- from 10% to 30% glutamic acid;
- from 5% to 25% arginine;
- from 5% to 20% aspartic acid;
- from 1% to 10% leucine;
- from 1% to 8% glycine;
the percentages being expressed by weight relative to the total weight of amino acids contained in the extract.

12. Method for preparing a product derived from a guar protein extract according to any one of the preceding claims,
comprising the following steps:
a) preparing a guar protein extract;
b) graft reaction onto functions of amino acids contained in the protein extract; and optionally
c) recovering the product obtained.

13. Use of the product according to any one of Claims 1 to 11, in:
- cosmetic compositions,
- compositions for household care,
- coating compositions, such as paints,
- food compositions for feeding humans,
- lubrication compositions,
- fluids used in the exploitation of petroleum or gas deposits,
- phytosanitary compositions,
- water treatments.

14. Use according to the preceding claim, as:
- surface-treating or surface-modifying agent,
- dispersant,
- emulsifier,
- sticking agent, anti-leaching agent, anti-drift agent, for phytosanitary formulations,
- adhesion agent,
- flocculant,
- surfactant,
- foam property modifying agent,
- foam stabiliser,
- coating appearance modifying agent, and/or
- viscosifying agent.

15. Pharmacological composition comprising the product according to any one of Claims 1 to 11.
